# FASCICULE DE BREVET EUROPEEN

(11) **EP 3 164 383 B1**
(45) Date de publication et mention de la délivrance du brevet: **21.11.2018**
(21) Numéro de dépôt: 15756958.3
(22) Date de dépôt: 01.07.2015
(51) Int. Cl.: C07C 333/16

(54) **PROCÉDÉ DE PRÉPARATION DE DITHIOCARBAMATES MÉTALLIQUES**
VERFAHREN ZUR HERSTELLUNG VON METALLDITHIOCARBAMATEN
METHOD FOR PRODUCING METAL DITHIOCARBAMATES

(30) Priorité: 03.07.2014 FR 1456370
(43) Date de publication de la demande: 10.05.2017
(73) Titulaire: MLPC International, 40370 Rion-Des-Landes (FR)
(72) Inventeur: AUTAA, Thierry, 64170 Artix (FR); AUBERT, Thierry, 64230 Lescar (FR)
(74) Mandataire: Albani, Dalila
(86) Numéro de dépôt international: PCT/FR2015/051810
(87) Numéro de publication internationale: WO 2016/001582

(56) Documents cités:
- EP-A2- 1 142 869
- US-A- 2 258 847
- US-A- 2 406 960
- DATABASE WPI Week 201440 Thomson Scientific, London, GB; AN 2014-L83972 XP002736558, & CN 103 755 613 A (SHANDONG SUNSINE CHEMICAL) 30 avril 2014 (2014-04-30)

## Description

L'invention concerne la fabrication de composés de type dithiocarbamates métalliques, agents connus comme accélérateurs de vulcanisation et utilisés couramment pour la vulcanisation de divers types de caoutchoucs. L'invention concerne en particulier un procédé de fabrication de dithiocarbamates de zinc à partir des dithiocarbamates de sodium correspondant et d'un sel de zinc inorganique.

Les dithiocarbamates de zinc sont très utilisés dans l'industrie du caoutchouc, du fait de leurs excellentes performances, que ce soit du point de vue de la cinétique de vulcanisation ou des propriétés physiques des caoutchoucs vulcanisés au moyen desdits dithiocarbamates.

Cependant, l'un des problèmes auquel doit faire face l'industrie du caoutchouc est la formation de nitrosamines cancérigènes volatiles au cours de la vulcanisation lors de l'utilisation de la plupart des dithiocarbamates de zinc classiques tels que les diméthyl-, diéthyl- ou dibutyl-dithiocarbamates de zinc.

Afin de pallier ce problème, l'une des solutions la plus utilisée actuellement est de remplacer les dithiocarbamates mentionnés ci-dessus par le dibenzyldithiocarbamate de zinc, encore identifié par son acronyme ZBEC (ou ZBDC ou ZTC). En effet, le ZBEC génère une nitrosamine non volatile à laquelle les travailleurs ne sont donc pas exposés, d'où la dénomination anglo-saxonne de « nitrosamine-safe dithiocarbamate » pour cet accélérateur de vulcanisation.

Ces accélérateurs de vulcanisation, qui sont sous forme de poudre, doivent être incorporés dans le caoutchouc solide, par exemple à l'aide d'un mélangeur à cylindres, afin que la dispersion de cette poudre soit la plus homogène possible dans la matrice caoutchouc, ceci pour assurer une vulcanisation homogène du caoutchouc et des propriétés physico-chimiques optimales.

Plus les poudres à disperser ont une granulométrie régulière et une taille de particules petite, meilleure est la dispersion. Par ailleurs, plus la granulométrie du produit est petite, plus le procédé de fabrication du produit est délicat et ceci pour deux raisons principales : au cours de la cristallisation (ou précipitation) du produit, généralement en phase aqueuse, la formation de très petites particules génère des problèmes d'agitation pouvant aller jusqu'à la formation d'un gel impossible à agiter ; d'autre part, les étapes de finition, à savoir filtration et séchage du produit sont d'autant plus délicates que la taille des particules est petite. Il peut s'avérer en particulier très long et très difficile (besoin de fortes pressions) de filtrer un produit dont la granulométrie moyenne est de l'ordre du micromètre en volume.

Il y a donc un compromis à trouver concernant la taille des particules du produit pour satisfaire ces deux objectifs contradictoires : pouvoir disposer d'un procédé de fabrication industriel techniquement et économiquement viable et disperser au mieux le produit fini dans le caoutchouc avant d'effectuer la vulcanisation. Pour la plupart des accélérateurs de vulcanisation, ce compromis se situe généralement pour une granulométrie moyenne en volume comprise entre 10 µm et 60 µm, gamme de la valeur moyenne (D50 ou D(4,3)) des particules mesurées avec les techniques bien connues de l'homme du métier, telles que par exemple avec un granulomètre laser.

Il est parfois difficile d'atteindre de telles valeurs de taille de particules car certains dithiocarbamates, en particulier certains dithiocarbamates de zinc, tels que par exemple les diméthyl- et dibenzyl-dithiocarbamates de zinc, cristallisent naturellement sous la forme de très petites particules, typiquement avec une valeur moyenne de taille des particules largement inférieures à 10 µm et plus généralement entre 2 µm et 5 µm en volume.

Le brevet US2406960 exemplifie l'utilisation d'agents tampons organiques (acétate, formiate et oxalate de sodium), pour la fabrication de dithiocarbamates de zinc ou de cadmium (sans mention du dibenzyldithiocarbamate de zinc) de granulométrie uniforme (sans mention de la valeur de granulométrie moyenne) adaptée à l'utilisation dans le caoutchouc.

Le problème du procédé décrit dans le brevet US2406960 est que l'utilisation d'agents tampons organiques génère une charge carbonée polluante très élevée dans les effluents. En effet, les agents tampons utilisés lors de la synthèse doivent être utilisés en quantités importantes pour être suffisamment efficaces (par exemple préférentiellement de l'ordre de 6% à 15% en poids par rapport au dithiocarbamate de sodium soluble) et se retrouvent intégralement dans les effluents aqueux qui sont rejetés dans l'environnement.

Le brevet US2258847 décrit un procédé de fabrication de dithiocarbamates métalliques particuliers, sans mention de la valeur de granulométrie moyenne, qui sont des dérivés d'amines secondaires dont l'un des radicaux au moins est un aromatique, contrairement au ZBEC, grâce à l'utilisation d'un agent tampon de type sel d'acide faible, tel que l'acétate de sodium, le borax ou un hydrogénocarbonate.

Le problème du procédé décrit dans le brevet US2258847 est que la synthèse est effectuée dans un solvant, tel qu'un alcool, ce qui complique significativement le procédé de préparation sur le plan industriel si l'on considère les nombreux problèmes rencontrés, et par exemple les problèmes liés à l'hygiène et à la sécurité, la nécessité de recycler le solvant, souvent en le purifiant par des techniques coûteuses telles que la distillation, et autres.

Le document EP1142869 décrit quant à lui un procédé de fabrication de dithiocarbamates de zinc, dont le ZBEC, conduisant à une granulométrie moyenne très élevée (de l'ordre de 100 µm, déterminée par microscopie) donc théoriquement facile à fabriquer, mais qui nécessite obligatoirement une étape de broyage supplémentaire pour l'utilisation dans le caoutchouc, comme indiqué précédemment.

Les problèmes du procédé décrit dans le document EP1142869 sont que ce procédé d'une part réclame une étape ultérieure de broyage afin d'atteindre une granulométrie suffisamment fine pour une dispersion satisfaisante dans le caoutchouc, et d'autre part ne semble pas conduire à une pureté satisfaisante de l'accélérateur de vulcanisation pour une application appropriée dans le caoutchouc.

Il reste donc un besoin pour un procédé de préparation de dithiocarbamates métalliques qui s'affranchit des problèmes rencontrés dans les procédés connus de l'art antérieur.

Ainsi, un premier objectif de la présente invention est la mise à disposition d'un procédé de préparation de dithiocarbamates métalliques conduisant directement à un produit sous forme de poudre de granulométrie adaptée, typiquement comprise entre 10 µm et 60 µm, avantageusement entre 10 µm et 40 µm, mieux encore entre 10 µm et 30 µm, bornes incluses, sans étape supplémentaire coûteuse de broyage.

Un autre objectif de la présente invention réside dans la mise à disposition d'un procédé de préparation de dithiocarbamates métalliques, qui ne génère pas de charge carbonée polluante due à l'agent tampon utilisé, dans les effluents issus dudit procédé de préparation, typiquement une demande chimique en oxygène (DCO) inférieure ou égale à 10 g/L d'effluents, généralement comprise entre 2 g/L et 6 g/L, plus généralement entre 3 g/L et 5 g/L.

La demande chimique en oxygène (ou DCO) est définie comme la consommation en dioxygène par les oxydants chimiques forts pour oxyder les substances organiques et minérales de l'eau. Elle permet d'évaluer la charge polluante des eaux usées, comme indiqué plus loin.

Un autre objectif est la mise à disposition d'un procédé de préparation de dithiocarbamates métalliques mettant en oeuvre un agent tampon bon marché et peu toxique, voire non toxique pour l'homme et pour l'environnement.

Un autre objectif encore est la mise à disposition d'un procédé de préparation de dithiocarbamates métalliques conduisant à un produit de grande pureté, typiquement une pureté supérieure ou égale à 97% en poids, ou tout au moins de pureté satisfaisante pour l'application principale envisagée comme accélérateur de vulcanisation, notamment pour les caoutchoucs.

L'obtention d'un dithiocarbamate métallique présentant une très bonne apparence visuelle, et notamment d'un dithiocarbamate métallique de couleur blanche, représente encore un autre objectif de la présente invention. D'autres objectifs encore apparaîtront dans la description de l'invention qui suit.

Il a maintenant été découvert que les objectifs précités peuvent être atteints en totalité ou au moins en partie, grâce au procédé de la présente invention qui met en oeuvre un agent tampon inorganique peu toxique, voire non toxique, et dont la valeur de pKa est strictement inférieure à 3, et plus particulièrement dont la valeur de pKa est strictement supérieure à -2 et strictement inférieure à 3, de préférence strictement supérieure à 0 et strictement inférieure à 3, de préférence encore strictement supérieure à 0,5 et strictement inférieure à 2,5. Grâce à l'utilisation de cet agent tampon inorganique, le procédé de l'invention ne génère pas ou peu de charge carbonée polluante dans les effluents aqueux.

Les valeurs de pKa au sens de la présente invention sont telles que définies dans l'ouvrage de J. March, « Advanced Organic Chemistry », 4e édition, (1992), pages 250-251).

Ainsi, et selon un premier aspect, la présente invention concerne l'utilisation d'un agent tampon inorganique dont la valeur de pKa est strictement inférieure à 3, et plus particulièrement dont la valeur de pKa est strictement supérieure à -2 et strictement inférieure à 3, de préférence strictement supérieure à 0 et strictement inférieure à 3, de préférence encore strictement supérieure à 0,5 et strictement inférieure à 2,5, pour la préparation d'un dithiocarbamate métallique, et plus particulièrement pour la préparation d'un dithiocarbamate métallique à partir d'un dithiocarbamate soluble, dans l'eau, dans un solvant hydro-organique ou un solvant organique, de préférence dans l'eau.

Par « agent tampon », on entend les composés communément utilisés par l'homme du métier dans les procédés de synthèse où il est avantageux, voire nécessaire de maintenir constant le pH du milieu réactionnel. Par agent tampon « inorganique », on entend les agents tampons ne contenant pas d'atome de carbone.

Des exemples non limitatifs des agents tampons inorganiques qui peuvent être utilisés dans le cadre de la présente invention comprennent les hydrogéno-sulfates (pKa = 2) de métaux alcalins, de préférence l'hydrogénosulfate de sodium et l'hydrogénosulfate de potassium, ou l'hydrogénosulfate d'ammonium, pour ne citer que les agents tampons aisément disponibles dans le commerce et qui permettent une conduite économique de la production de dithiocarbamates métalliques sur le plan industriel.

Parmi ces agents tampons inorganiques, on préfère tout particulièrement les hydrogénosulfates de métaux alcalins, plus particulièrement les hydrogénosulfates de sodium ou de potassium, et de manière tout à fait préférée l'hydrogénosulfate de sodium.

Il a été découvert, de façon tout à fait surprenante, que l'utilisation d'un agent tampon inorganique dont le pKa est strictement inférieur à 3 pour la préparation d'un dithiocarbamate métallique à partir de dithiocarbamate soluble dans l'eau permet une bonne agitation pendant la synthèse et conduit à une filtration et un séchage aisés.

Selon un autre aspect, la présente invention concerne le procédé de préparation d'un dithiocarbamate métallique, comprenant au moins les étapes suivantes :
a) préparation d'une solution d'un dithiocarbamate dans l'eau, dans un solvant hydro-organique ou dans un solvant organique,
b) préparation d'une solution d'un cation métallique Mⁿ⁺ dans l'eau, dans un solvant hydro-organique ou dans un solvant organique,
c) ajout d'un agent tampon inorganique de pKa strictement inférieur à 3, de préférence dont le pKa est définit par -2 < pKa < 3, de préférence encore 0 < pKa < 3, avantageusement 0,5 < pKa < 2,5, dans l'une au l'autre des solutions préparées dans les étapes a) et/ou b),
d) mise en contact des deux solutions préparées dans les étapes précédentes, provoquant la réaction entre le dithiocarbamate soluble et le cation métallique Mⁿ⁺ et l'apparition de cristaux du sel métallique de dithiocarbamate,
e) étape de finition de la réaction, sous agitation, pendant une durée comprise entre quelques minutes, voire quelques dizaines de minutes à quelques heures,
f) filtration, séchage et récupération dudit sel métallique de dithiocarbamate.

Dans la présente invention, on entend par « dithiocarbamate » un sel d'acide dithiocarbamique. Par « acide dithiocarbamique », on entend le composé de formule (RR')N-C(=S)-S-H, dans laquelle R et R', identiques ou différents sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, ou bien R et R' forment ensemble, et avec l'atome d'azote auquel ils sont reliés, un radical hétérocyclique à 5, 6, 7 ou 8 chaînons.

Parmi les radicaux hydrocarbonés saturés ou totalement ou partiellement insaturés, linéaires, ramifiés ou cycliques, contenant de 1 à 30 atomes de carbone, on peut citer à titre d'exemples non limitatifs le radicaux méthyle, éthyle, et les radicaux linéaires ou ramifiés propyles, butyles, pentyles, hexyles, heptyles, octyles, nonyles, décyles, undécyles, dodécyles, chaînes hydrocarbonées grasses saturées ou insaturées, phényle, benzyle, phénéthyle, naphtyle, et autres.

Des exemples d'acides dithiocarbamiques comprennent, de manière non limitative, l'acide dibenzyldithiocarbamique (R = R' = benzyle), l'acide pentaméthylène dithiocarbamique (R et R' formant ensemble le radical divalent -CH₂(CH₂)₃CH₂-), l'acide hexa-méthylène dithiocarbamique (R et R' formant ensemble le radical divalent -CH₂(CH₂)₄CH₂-), l'acide diamyldithiocar-bamique, l'acide di-(2-éthylhexyl)dithiocarbamique, l'acide di-iso-nonyldithiocarbamique, l'acide di-cocodithiocarbamique, l'acide di-oléyldithiocarbamique l'acide ditallow-dithiocarbamique. Les acides dithiocarbamiques préférés dans le cadre de la présente invention sont ceux pour lesquels R et R' représentent chacun un radical hydrocarboné tel que défini ci-dessus et de préférence encore ceux pour lesquels R et R' sont identiques.

Le procédé de la présente invention est particulièrement adapté à la préparation des sels métalliques d'acide dithiocarbamique, à partir de tout sel connu d'acide dithiocarbamique soluble dans l'eau. Le procédé est tout particulièrement adapté à la préparation des sels métalliques d'acides N-aralkyldithiocarbamiques, plus particulièrement des sels métalliques d'acides N-diaralkyldithiocarbamiques. Parmi ces sels, préférence est donnée aux sels faiblement basiques, comme par exemple les sels métalliques de l'acide dibenzyldithiocarbamique.

Le dithiocarbamate mis en oeuvre à l'étape a) est un sel d'acide dithiocarbamique, ledit sel étant soluble. Par « soluble », on entend au sens de la présente invention le fait qu'un composé est soluble dans l'eau, mais aussi soluble dans les solvants hydro-organiques, et/ou les solvants organiques, de préférence les solvants polaires, parmi lesquels on peut citer notamment les alcools, les glycols, le diméthylsulfoxyde, le dioxane, et autres, par exemple l'éthanol, avec une solubilité comprise entre 5% et 50%, bornes incluses, exprimée en poids de composé par rapport à l'ensemble composé + solvant, de préférence entre 10% et 30%, bornes incluses, de préférence encore entre 20% et 30%, bornes incluses.

Des exemples de sels d'acide dithiocarbamique soluble qui peuvent être mis en oeuvre dans le cadre de la présente invention, comprennent, de manière non limitative, les sels d'alcalins ou d'alcalino-terreux, notamment les sels de sodium, potassium, calcium, baryum mais aussi les sels d'ammonium et les sels d'amines. Parmi ces sels on préfère en particulier les sels d'alcalins et tout particulièrement les sels de sodium ou de potassium.

Comme indiqué précédemment, les sels d'acides dithiocarbamiques solubles mis en oeuvre à l'étape a) sont soit connus et disponibles dans le commerce, soit facilement préparés à partir de modes opératoires connus et accessibles dans les brevets, la littérature scientifique, les « Chemical Abstracts » ou encore l'Internet.

À titre d'exemples non limitatifs, les sels d'acide dithiocarbamique solubles peuvent aisément être obtenus à partir d'amines secondaires et de sulfure de carbone (CS₂) en présence d'une solution aqueuse, hydro-organique ou organique, d'hydroxyde de sodium, d'hydroxyde de potassium, d'hydroxyde de baryum, d'hydroxyde d'ammonium ou encore d'ammoniac.

De préférence, le sel d'acide dithiocarbamique est mis en solution, de préférence dans un solvant aqueux, de préférence encore dans l'eau, dans des proportions appropriées, comme indiqué par exemple dans « The dithiocarbamates and related compounds », G. D. Thorn et R. A. Ludwig, (1962), Elsevier Monographs, chapitre 2).

En règle générale, la concentration en sel d'acide dithiocarbamique soluble est d'environ 10% en poids, par rapport au poids total du milieu dans lequel il est mis en solution. Un des avantages du procédé selon l'invention est qu'il est possible de mettre en oeuvre des solutions plus concentrées, typiquement en concentrations comprises entre 10% et 30% en poids, bornes incluses, et de préférence encore entre 20% et 30% en poids, par rapport au poids total de la solution. Il doit être compris que cette concentration ne peut excéder la limite de solubilité du sel d'acide dithiocarbamique dans le solvant considéré.

La quantité d'agent tampon est généralement comprise entre environ 0,5% et environ 20% par rapport au poids du dithiocarbamate mis en oeuvre. On préfère utiliser une quantité d'agent tampon comprise entre environ 0,8% et environ 10% en poids par rapport au poids du dithiocarbamate mis en oeuvre, et de préférence encore entre 1% et 5% en poids par rapport au poids du dithiocarbamate mis en oeuvre, les intervalles de valeurs s'entendant bornes incluses.

Le cation métallique Mⁿ⁺ est mélangé à un agent tampon tel que défini précédemment, de préférence sous agitation, selon tout moyen connu en soi par l'homme du métier. Par exemple et selon un aspect préféré de l'invention le cation métallique Mⁿ⁺ est apporté sous forme de sel métallique préparé à partir d'un métal M et d'au moins un acide fort, de préférence un acide minéral fort, par exemple et de manière non limitative choisi parmi l'acide sulfurique, l'acide chlorhydrique, l'acide bromhydrique et l'acide nitrique, de préférence parmi l'acide sulfurique et l'acide chlorhydrique. Des sels métalliques obtenus à partir d'acides faibles peuvent également être envisagés.

Les sels métalliques utilisés sont des sels de métaux lourds, tels que ceux habituellement utilisés en tant qu'agents de précipitation pour la préparation de sels métalliques stables d'acides dithiocarbamiques. Ces sels sont avantageusement des sels métalliques présentant une bonne solubilité dans le milieu solvant envisagé (eau, solvant organique ou système eau/solvant organique), c'est-à-dire une solubilité permettant une concentration en solution des ions métalliques Mⁿ⁺ du même ordre de grandeur que la concentration molaire en sel d'acide dithiocarbamique soluble. Dans cette configuration, et sans être toutefois lié par la théorie, on pense que la cinétique de précipitation du dithiocarbamate métallique final est suffisamment rapide pour générer un grand nombre de cristaux de petite taille. Généralement, on observe alors que les cristaux de dithiocarbamate métallique apparaissent au furet à mesure de la mise en contact des deux solutions préparées dans les étapes a) et b).

De tels sels de métaux lourds sont bien connus de l'homme du métier. Des exemples non limitatifs de sels de métaux lourds qui peuvent être avantageusement utilisés dans le cadre de la présente invention sont les sels de zinc, nickel, cuivre, manganèse, molybdène, palladium, arsenic, tellure, chrome, cadmium, mercure, plomb, fer et autres. Le procédé de la présente invention est tout particulièrement adapté pour la préparation de sels de zinc d'acides dithiocarbamiques.

Le nombre de moles de sel métallique Mⁿ⁺ correspond à la stoechiométrie désirée, selon le nombre de moles de sel d'acide dithiocarbamique soluble dans l'eau, le solvant organique ou le solvant hydro-organique. En règle générale le nombre de moles de sel d'acide dithiocarbamique soluble correspond à environ n fois le nombre de moles de cation Mⁿ⁺. Il doit en outre être compris que la concentration molaire du sel comportant le cation Mⁿ⁺ dépend de sa solubilité dans l'eau, dans le solvant organique ou dans le solvant hydro-organique. En règle générale, la concentration molaire en cation métallique Mⁿ⁺ optimale dépend de plusieurs facteurs dont la concentration finale désirée du sel métallique d'acide dithiocarbamique, la température de la réaction ou la productivité désirée du procédé. Par concentration molaire, on entend le nombre de moles par litre de solution.

À titre d'exemple non limitatif, dans le cas du cation Zn²⁺, celui-ci est apporté par le sulfate de zinc (ou l'un de ses hydrates) en solution dans le mélange eau et agent tampon, en concentration molaire comprise entre 0,1 et 4 moles par litre de solution. Comme indiqué précédemment, la concentration molaire en sel métallique doit avantageusement être du même ordre de grandeur que la concentration molaire en sel d'acide dithiocarbamique.

Le cation métallique Mⁿ⁺ peut être mis en solution dans l'eau, dans un solvant hydro-organique ou dans un solvant organique, et on préfèrera utiliser comme milieu solvant pour le cation métallique un milieu solvant de nature semblable ou identique à celui utilisé pour solubiliser le dithiocarbamate (étape a). Selon un aspect tout particulièrement préféré, le cation métallique Mⁿ⁺ est mis en solution dans l'eau. On préfère ainsi tout particulièrement mettre en oeuvre le procédé de l'invention avec une solution de dithiocarbamate dans l'eau et une solution de sel métallique dans l'eau.

L'agent tampon inorganique (étape c) peut être ajouté en totalité dans la solution de dithiocarbamate préparée à l'étape a) ou bien en totalité dans la solution de sel métallique préparée à l'étape b). En variante, on peut ajouter une partie de l'agent tampon inorganique dans la solution de dithiocarbamate et le restant de l'agent tampon inorganique dans la solution de sel métallique.

Comme indiqué précédemment, la solution obtenue à l'étape a) est mise en contact avec la solution obtenue à l'étape b), contenant de préférence l'agent tampon inorganique. Dans un mode de réalisation préféré, la solution obtenue à l'étape a) est ajoutée progressivement, en quelques heures, dans la solution de sel métallique contenant l'agent tampon inorganique.

Cette mise en contact, sous agitation, des solutions des étapes a) et b) avec l'agent tampon inorganique de l'étape c), conduit à la précipitation (ou cristallisation) (étape d) du sel métallique d'acide dithiocarbamique attendu. L'étape d) décrite plus haut est suivie d'une phase de finition, opérée sous agitation (étape e), cette phase de finition comprenant éventuellement une phase de croissance des cristaux.

L'agitation peut être obtenue au moyen de tout type d'agitateur connu de l'homme du métier, et par exemple et de manière non limitative, au moyen d'un agitateur à pales, de préférence d'un agitateur multi-pales, par exemple tripales. La vitesse d'agitation dépend de nombreux facteurs, dont par exemple la concentration et la température du milieu.

En règle générale, la vitesse d'agitation est comprise entre environ 100 tours/min et environ 700 tours/min, de préférence entre environ 300 tours/min et environ 500 tours/min, bornes incluses. Dans le cas d'un agitateur à pales, la vitesse d'agitation en bout de pale est généralement comprise entre environ 0,4 m.s⁻¹ et environ 4,5 m.s⁻¹, de préférence entre environ 1,2 m.s⁻¹ et environ 3 m.s⁻¹, bornes incluses.

Le procédé de l'invention peut être réalisé à toute température. Pour des raisons évidentes de facilités de mise en oeuvre, on préfère toutefois réaliser le procédé dans des gammes de températures où les différents réactifs, solutions et milieux réactionnels sont à l'état liquide. On peut en outre prévoir de chauffer légèrement les solutions afin de faciliter et/ou augmenter la solubilité des sels. Ainsi par exemple, les températures qui peuvent être mises en oeuvre dans les étapes du procédé de l'invention, peuvent être comprises entre 0°C et la température d'ébullition du CS₂ pour l'étape a) et, pour les étapes b) à f) entre 0°C et la température d'ébullition du solvant, de préférence entre la température ambiante et la température d'ébullition du solvant, de préférence encore entre 30°C et 60°C, de préférence encore entre 40°C et 55°C, mieux encore entre 45°C et 50°C.

Le procédé selon l'invention est généralement réalisé à pression atmosphérique, mais on ne sortirait pas du cadre de l'invention si le procédé était opéré sous légère dépression ou sous légère pression. Le procédé de l'invention n'implique en effet pas de phase gazeuse, de sorte que la pression appliquée au milieu réactionnel n'a que peu d'effet, voire est sans effet.

Les étapes de mise en contact d) et de finition e) sont rendues particulièrement aisées en raison de la présence de l'agent tampon spécifique décrit précédemment, qui permet :
- une agitation très facile du milieu réactionnel, sans phénomène de collage, d'épaississement ou de prise en masse ; ceci permet d'utiliser des solutions concentrées de dithiocarbamates solubles, typiquement entre 10 et 30%, voire entre 20 et 30%,
- une croissance des cristaux du sel métallique de l'acide dithiocarbamique tout à fait appropriée à l'obtention de cristaux de granulométrie correspondant à l'objectif à atteindre, c'est-à-dire une granulométrie comprise entre 10 µm et 60 µm en volume, bornes incluses, sans étape supplémentaire de broyage.

La granulométrie des sels métalliques d'acides dithiocarbamiques obtenus selon le procédé de la présente invention est mesurée à l'aide d'un granulomètre laser de type MALVERN Mastersizer 2000 (http://www.malvern.com/fr/products/ product-range/mastersizer-range/mastersizer-2000/default.aspx). Cet appareil permet d'analyser la granulométrie moyenne en volume D[4,3], exprimée en micromètres (µm).

Les cristaux obtenus à l'étape e) sont ensuite filtrés, selon toute méthode connue de l'homme du métier, et par exemple au moyen d'un filtre-presse, d'une essoreuse, ou d'une centrifugeuse. Les cristaux filtrés sont ensuite séchés selon toute méthode connue de l'homme du métier, avant d'être récupérés puis utilisés directement ou stockés après ensachage, ensilage et autres, sans opération supplémentaire de broyage.

Les sels métalliques d'acides dithiocarbamiques obtenus selon le procédé de la présente invention présentent, comme indiqué précédemment, une granulométrie tout à fait adaptée, sans qu'il soit nécessaire de procéder à un broyage supplémentaire, pour être utilisés directement en tant qu'accélérateurs de vulcanisation pour la vulcanisation de divers types de caoutchoucs, comme par exemple, à titre non limitatif, les caoutchoucs de type naturel, styrène-butadiène, nitrile et butyle (voir par exemple « Vulcanization and Vulcanizing agents », W. Hoffmann (1967), Maclaren, pages 106 et suivantes ou encore : « http://nocil.com/DTechnicalNote-Vulcanization-Dec10.pdf », page 8.

En outre, le procédé de la présente invention permet d'obtenir des sels métalliques d'acides dithiocarbamiques de grande pureté, typiquement supérieure à 96%, qui se caractérisent notamment par leur couleur blanche.

Enfin, le procédé de la présente invention met en oeuvre des agents tampons inorganiques permettant ainsi de limiter la DCO des effluents à des valeurs faibles, inférieures ou égales à 10 g/L d'effluents ou de l'ordre de 45 g/kg de dithiocarbamate métallique produit.

Il est rappelé ici que la DCO est une des mesures principales des effluents pour les normes de rejet. Celle-ci est généralement déterminée par colorimétrie, et dans le cadre de la présente invention, la DCO est déterminée au moyen d'un colorimètre de type Hach et de la méthode associée N°8000 (cf. « https://www.hach.com/asset-get.download.jsa?id=7639982260 », page 198 sqq.

Exprimée en g/kg de dithiocarbamate métallique produit, il a ainsi été observé que la DCO typique des effluents issus du procédé de la présente invention est en règle générale typiquement inférieure à une valeur de l'ordre de 45 g/kg, plus généralement comprise entre 9 g/kg et 27 g/kg, plus généralement encore entre 13 g/kg et 22 g/kg de dithiocarbamate métallique. Cette valeur de DCO dépend de la concentration finale en dithiocarbamate métallique et est exprimée à la fois par la concentration par litre d'effluent et par la quantité totale de DCO générée par kg de produit désiré.

Les exemples suivants illustrent l'invention sans la limiter.

**Exemple 1 :** (comparatif) **Préparation du dibenzyldithiocarbamate de zinc** (ZBEC) utilisant l'un des agents tampons décrits dans US2406960 :
l'acétate de sodium (agent tampon de pKa = 4,8).

Dans un réacteur de 2,5 litres en verre muni :
- d'un agitateur tripales (pales inclinées à 45°) de type Mixel de 77 mm de diamètre tournant à 400 tours/min.,
- d'une double enveloppe contenant un fluide caloporteur,
- d'une sonde de température,
- d'un réfrigérant alimenté par de l'eau à 15-20°C,
- d'un tube d'introduction relié à une pompe péristaltique et à un flacon contenant le dibenzyldithiocarbamate de sodium (NaDBC),
on ajoute 300 g d'une solution de sulfate de zinc à 22,6% en poids et 19,3 g (6% en poids par rapport à la solution de sulfate de zinc, soit 7,8 % par rapport au poids du dibenzyldithiocarbamate de sodium) d'acétate de sodium et on chauffe cette solution à 50°C.

À la solution précédente, on ajoute, toujours sous agitation et en 4,5 heures, 1000 g d'une solution aqueuse à 24,7% en poids de dibenzyldithiocarbamate de sodium (NaDBC), préparée selon l'une des méthodes bien connues de l'homme de l'art à partir de dibenzylamine, disulfure de carbone et NaOH.

La suspension blanche et bien agitable obtenue à la fin de l'addition est maintenue sous agitation à la température de 50°C pendant environ 2 heures puis filtrée sur un filtre fermé en inox sous 1 bar (100 kPa) de pression. La DCO (Demande Chimique en Oxygène, directement fonction de la charge carbonée oxydable, mesurée sur colorimètre Hach, méthode n° 8000, comme indiqué précédemment) du filtrat est de 13 g/L de filtrat soit 52 g/kg de ZBEC obtenu.

Après lavage du produit par ajout de 600 mL d'eau sur le filtre et séchage du produit à 90°C dans une étuve ventilée, on obtient 277 g d'une poudre blanche de 19 µm de diamètre moyen en volume, de point de fusion 187°C et de pureté 90%, ce qui correspond à un rendement en ZBEC de 98%.

### Exemple 2 : (selon l'invention) Préparation du dibenzyldithiocarbamate de zinc (ZBEC)

Dans un réacteur de 2,5 litres en verre muni :
- d'un agitateur tripales (pales inclinées à 45°) de type Mixel de 77 mm de diamètre tournant à 400 tours/min.,
- d'une double enveloppe contenant un fluide caloporteur,
- d'une sonde de température,
- d'un réfrigérant alimenté par de l'eau à 15-20°C,
- d'un tube d'introduction relié à une pompe péristaltique et à un flacon contenant le dibenzyldithiocarbamate de sodium (NaDBC),
on ajoute une solution de 81,6 g (23,5% en poids) de sulfate de zinc monohydraté et 5,4 g (1,6% en poids par rapport à la solution de sulfate de zinc, soit 2,2 % par rapport au poids du dibenzyldithiocarbamate de sodium) d'hydrogénosulfate de sodium dans 260 g d'eau et on chauffe cette solution à 50°C.

À la solution précédente, on ajoute, toujours sous agitation et en 4,5 heures, 1000 g d'une solution aqueuse à 24,3% en poids de dibenzyldithiocarbamate de sodium (NaDBC), préparée selon l'une des méthodes bien connues de l'homme de l'art à partir de dibenzylamine, disulfure de carbone et hydroxyde de sodium.

La suspension blanche et bien agitable obtenue à la fin de l'addition est maintenue sous agitation à la température de 50°C pendant environ 2 heures puis filtrée sur un filtre fermé en inox sous 1 bar (100 kPa) de pression. La DCO (Demande Chimique en Oxygène, directement fonction de la charge carbonée oxydable, mesurée sur colorimètre Hach, méthode n° 8000, comme indiqué précédemment) du filtrat est de 4 g/L soit 18 g/kg de ZBEC obtenu.

Après lavage du produit par ajout de 600 mL d'eau sur le filtre et séchage du produit à 90°C dans une étuve ventilée, on obtient 256 g d'une poudre blanche de 15 µm de diamètre moyen en volume, de point de fusion 185°C et de pureté 97%, ce qui correspond à un rendement en ZBEC de 98%.

L'agent tampon qui a donné de manière très surprenante les meilleurs résultats est l'hydrogénosulfate de sodium, dont le pKa est de 2. Ces résultats sont tout à fait inattendus et surprenants au regard de l'enseignement du document US2406960, mais conduisant aux mêmes effets (milieu réactionnel très facilement agitable, granulométrie du produit satisfaisante, bonne qualité du produit final) que les agents tampons décrits dans l'art antérieur, et notamment dans le document US2406960, dont les pKa sont compris strictement entre 3 et 6, selon les auteurs.

Il a ainsi été constaté que l'utilisation d'un agent tampon inorganique, l'hydrogénosulfate de sodium, permet non seulement d'obtenir un rendement en ZBEC tout à fait comparable au rendement obtenu avec un agent tampon organique, l'acétate de sodium, mais aussi un produit final, ZBEC, de pureté plus grande et avec un impact environnemental des effluents rejetés remarquablement réduite de l'ordre de 65 à 70 %.

Enfin, la quantité d'agent tampon inorganique nécessaire est très faible, de l'ordre de quelques % par rapport au dithiocarbamate soluble, ce qui n'impacte pas l'économie globale du procédé.

### Exemple 3 : (comparatif) Préparation du dibenzyldithiocarbamate de zinc (ZBEC) selon le mode opératoire décrit dans EP1142869.

Dans un réacteur de 2,5 litres en verre muni :
- d'un agitateur tripales (pales inclinées à 45°) de type Mixel de 77 mm de diamètre tournant à 550 tours/min.,
- d'une double enveloppe contenant un fluide caloporteur,
- d'une sonde de température,
- d'un réfrigérant alimenté par de l'eau à 15-20°C,
on ajoute 256 g de dibenzylamine, 260 g d'eau, et 0,7 g de N-dodécylpyrrolidone.

À la solution précédente, on ajoute sous agitation, à 20°C et en 15 minutes, une solution de 187,2 g de sulfate de zinc heptahydraté dans 1040 g d'eau. Un précipité blanchâtre apparaît au cours de l'addition.

On ajoute ensuite, toujours à 20°C, 102 g de CS₂ en 1,5 h puis on chauffe le milieu réactionnel 35 minutes à 33°C. Enfin, on neutralise le milieu réactionnel par ajout, en 40 minutes, d'une solution de 52 g d'hydroxyde de sodium dans 650 g d'eau : la température du milieu passe de 33°C à 37°C. Après 1 heure d'agitation à 36-38°C, on filtre la suspension sur un filtre fermé en inox sous 1 bar (100 kPa) de pression. La DCO (Demande Chimique en Oxygène, directement fonction de la charge carbonée oxydable, mesurée sur colorimètre Hach, méthode n° 8000, comme indiqué précédemment) du filtrat est de 1,6 g/L de filtrat soit 8,4 g/kg de ZBEC.

On lave ensuite le solide sur le filtre avec 3 litres d'eau à 80°C avant de le sécher dans une étuve sous vide (10 mbar ; 1 kPa) à 50°C. On récupère 284 g d'un solide blanchâtre contenant de l'ordre de 15% de dibenzylamine (déterminée par RMN) de 66 µm de granulométrie moyenne et présentant un très faible point de fusion à 167°C, caractéristique d'une pureté faible, ne convenant pas pour les applications habituelles dans les caoutchoucs, pour lesquelles le point de fusion exigé est généralement supérieur à 180°C. Le rendement en ZBEC obtenu est de 82%.

## Revendications

1. Utilisation d'un agent tampon inorganique dont la valeur de pKa est strictement inférieure à 3, et plus particulièrement dont la valeur de pKa est strictement supérieure à -2 et strictement inférieure à 3, de préférence strictement supérieure à 0 et strictement inférieure à 3, de préférence encore strictement supérieure à 0,5 et strictement inférieure à 2,5, pour la préparation d'un dithiocarbamate métallique, et plus particulièrement pour la préparation d'un dithiocarbamate métallique à partir d'un dithiocarbamate soluble, dans l'eau, dans un solvant hydro-organique ou un solvant organique, de préférence dans l'eau.

2. Utilisation selon la revendication 1, dans laquelle l'agent tampon inorganique est choisi parmi les hydrogéno-sulfates de métaux alcalins ou d'ammonium, de préférence l'agent tampon inorganique est l'hydrogénosulfate de sodium ou l'hydrogénosulfate de potassium.

3. Procédé de préparation d'un dithiocarbamate métallique, comprenant au moins les étapes suivantes :
a) préparation d'une solution d'un dithiocarbamate dans l'eau, dans un solvant hydro-organique ou dans un solvant organique,
b) préparation d'une solution d'un cation métallique Mⁿ⁺ dans l'eau, dans un solvant hydro-organique ou dans un solvant organique,
c) ajout d'un agent tampon inorganique de pKa strictement inférieur à 3, de préférence dont le pKa est définit par -2 < pKa < 3, de préférence encore 0 < pKa < 3, avantageusement 0,5 < pKa < 2,5, dans l'une au l'autre des solutions préparées dans les étapes a) et/ou b),
d) mise en contact des deux solutions préparées dans les étapes précédentes, provoquant la réaction entre le dithiocarbamate soluble et le cation métallique Mⁿ⁺ et l'apparition de cristaux du sel métallique de dithiocarbamate,
e) étape de finition de la réaction, sous agitation, pendant une durée comprise entre quelques minutes, voire quelques dizaines de minutes à quelques heures,
f) filtration, séchage et récupération dudit sel métallique de dithiocarbamate.

4. Procédé selon la revendication 3, dans lequel la concentration en sel d'acide dithiocarbamique est comprise entre 10% et 30% en poids, bornes incluses, et de préférence encore entre 20% et 30% en poids, par rapport au poids total de la solution.

5. Procédé selon la revendication 3 ou la revendication 4, dans lequel la quantité d'agent tampon est comprise entre environ 0,5% et environ 20% par rapport au poids du dithiocarbamate soluble mis en oeuvre, de préférence entre environ 0,8% et environ 10% en poids par rapport au poids du dithiocarbamate mis en oeuvre, et de préférence encore entre 1% et 5% en poids par rapport au poids du dithiocarbamate mis en oeuvre, bornes incluses.

6. Procédé selon l'une quelconque des revendications 3 à 5, dans lequel sont mis en oeuvre une solution de dithiocarbamate dans l'eau et une solution de sel métallique dans l'eau.

7. Procédé selon l'une quelconque des revendications 3 à 5, dans lequel la solution obtenue à l'étape a) est mise en contact avec la solution obtenue à l'étape b), contenant de préférence l'agent tampon inorganique, et de préférence la solution obtenue à l'étape a) est ajoutée progressivement, en quelques heures, dans la solution de sel métallique contenant l'agent tampon inorganique.

## Patentansprüche

1. Verwendung eines anorganischen Puffermittels, dessen pKa-Wert streng kleiner als 3 ist und dessen pKa-Wert spezieller streng größer als -2 und streng kleiner als 3, vorzugsweise streng größer als 0 und streng kleiner als 3, weiter bevorzugt streng größer als 0,5 und streng kleiner als 2,5 ist, zur Herstellung eines Metalldithiocarbamats und spezieller zur Herstellung eines Metalldithiocarbamats aus einem löslichen Dithiocarbamat in Wasser, in einem wässrig-organischen Lösungsmittel oder einem organischen Lösungsmittel, vorzugsweise in Wasser.

2. Verwendung nach Anspruch 1, wobei das anorganische Puffermittel aus Alkalimetall- oder Ammoniumhydrogensulfaten ausgewählt ist und es sich bei dem organischen Puffermittel vorzugsweise um Natriumhydrogensulfat oder Kaliumhydrogensulfat handelt.

3. Verfahren zur Herstellung eines Metalldithiocarbamats, das mindestens die folgenden Schritte umfasst:
a) Herstellung einer Lösung eines Dithiocarbamats in Wasser, in einem wässrigen-organischen Lösungsmittel oder in einem organischen Lösungsmittel,
b) Herstellung einer Lösung eines Metallkations Mⁿ⁺ in Wasser, in einem wässrig-organischen Lösungsmittel oder in einem organischen Lösungsmittel,
c) Zugabe eines anorganischen Puffermittels, dessen pKa-Wert streng kleiner als 3 ist und dessen pKa-Wert vorzugsweise durch -2 < pKa < 3, weiter bevorzugt 0 < pKa < 3, vorteilhafterweise 0,5 < pKa < 2,5 definiert ist, zu der einen oder der anderen der in den Schritten a) und/oder b) hergestellten Lösungen,
d) Inkontaktbringen der beiden in den vorhergehenden Schritten hergestellten Lösungen zum Bewirken der Reaktion zwischen dem löslichen Dithiocarbamat und dem Metallkation Mⁿ⁺ und des Erscheinens von Kristallen des Dithioarbamat-Metallsalzes,
e) einen Reaktgionsfertigstellungsschritt unter Rühren über einen Zeitraum zwischen einigen Minuten oder einigen Dutzend Minuten bis zu einigen Stunden,
f) Filtration, Trocknung und Gewinnung des Dithiocarbamat-Metallsalzes.

4. Verfahren nach Anspruch 3, bei dem die Konzentration von Dithiocarbamidsäuresalz zwischen 10 und 30 Gew.-% einschließlich der Grenzen und vorzugsweise zwischen 20 und 30 Gew.-%, bezogen auf das Gesamtgewicht der Lösung, liegt.

5. Verfahren nach Anspruch 3 oder Anspruch 4, bei dem die Menge von Puffermittel zwischen ungefähr 0,5 % und ungefähr 20 %, bezogen auf das Gewicht des eingesetzten löslichen Dithiocarbamats, vorzugsweise zwischen ungefähr 0,8 und ungefähr 10 Gew.-%, bezogen auf das Gewicht des eingesetzten Dithiocarbamats, und weiter bevorzugt zwischen 1 und 5 Gew.-%, bezogen auf das Gewicht des eingesetzten Dithiocarbamats, einschließlich der Grenzen liegt.

6. Verfahren nach einem der Ansprüche 3 bis 5, bei dem eine Lösung von Dithiocarbamat in Wasser und eine Lösung von Metallsalz in Wasser eingesetzt werden.

7. Verfahren nach einem der Ansprüche 3 bis 5, bei dem die in Schritt a) erhaltene Lösung mit der in Schritt b) erhaltenen Lösung, die vorzugsweise das anorganische Puffermittel umfasst, in Kontakt gebracht wird und vorzugsweise die in Schritt a) erhaltene Lösung allmählich innerhalb von einigen Stunden zu der Lösung von Metallsalz, die das anorganische Puffermittel enthält, gegeben wird.

## Claims

1. Use of an inorganic buffer, the pKa value of which is strictly less than 3 and more particularly the pKa value of which is strictly greater than -2 and strictly less than 3, preferably strictly greater than 0 and strictly less than 3, more preferably strictly greater than 0.5 and strictly less than 2.5, for the preparation of a metal dithiocarbamate and more particularly for the preparation of a metal dithiocarbamate starting from a dithiocarbamate which is soluble in water, in an aqueous/organic solvent or an organic solvent, preferably in water.

2. Use according to Claim 1, in which the inorganic buffer is chosen from alkali metal or ammonium hydrogensulphate; preferably, the inorganic buffer is sodium hydrogensulphate or potassium hydrogensulphate.

3. Process for the preparation of a metal dithiocarbamate, comprising at least the following stages:
a) preparation of a solution of a dithiocarbamate in water, in an aqueous/organic solvent or in an organic solvent,
b) preparation of a solution of a metal cation Mⁿ⁺ in water, in an aqueous/organic solvent or in an organic solvent,
c) addition of an inorganic buffer with a pKa strictly of less than 3, the pKa of which is preferably defined by -2 < pKa < 3, or preferably 0 < pKa < 3, advantageously 0.5 < pKa < 2.5, to one or other of the solutions prepared in stages a) and/or b),
d) bringing the two solutions prepared in the preceding stages into contact, bringing about the reaction between the soluble dithiocarbamate and the metal cation Mⁿ⁺ and the appearance of crystals of the metal dithiocarbamate salt,
e) stage of finishing the reaction, with stirring, for a period of time of between a few minutes, indeed even several tens of minutes, to several hours,
f) filtration, drying and recovery of the said metal dithiocarbamate salt.

4. Process according to Claim 3, in which the concentration of dithiocarbamic acid salt is between 10% and 30% by weight, limits included, and more preferably between 20% and 30% by weight, with respect to the total weight of the solution.

5. Process according to Claim 3 or Claim 4, in which the amount of buffer is between approximately 0.5% and approximately 20%, with respect to the weight of the soluble dithiocarbamate employed, preferably between approximately 0.8% and approximately 10% by weight, with respect to the weight of the dithiocarbamate employed, and more preferably between 1% and 5% by weight, with respect to the weight of the dithiocarbamate employed, limits included.

6. Process according to any one of Claims 3 to 5, in which a solution of dithiocarbamate in water and a solution of metal salt in water are employed.

7. Process according to any one of Claims 3 to 5, in which the solution obtained in stage a) is brought into contact with the solution obtained in stage e), preferably containing the inorganic buffer, and, preferably, the solution obtained in stage a) is gradually added, over a few hours, to the solution of metal salt containing inorganic buffer.
